(19) Europäisches Patentamt / European Patent Office / Office européen des brevets

(11) **EP 4 768 035 A2**

## (12) EUROPEAN PATENT APPLICATION

(43) Date of publication:
**01.07.2026 Bulletin 2026/27**

(21) Application number: **26173067.5**

(22) Date of filing: **29.12.2021**

(51) International Patent Classification (IPC):
**A61K 35/741** (2015.01)

(52) Cooperative Patent Classification (CPC):
**A61K 35/742; A61P 31/00; C12N 1/20;
C12N 1/205;** A23L 33/135; C12R 2001/01;
C12R 2001/25

(84) Designated Contracting States:
**AL AT BE BG CH CY CZ DE DK EE ES FI FR GB
GR HR HU IE IS IT LI LT LU LV MC MK MT NL NO
PL PT RO RS SE SI SK SM TR**

(30) Priority: **05.01.2021 DK PA202100013
29.04.2021 DK PA202100433**

(62) Document number(s) of the earlier application(s) in
accordance with Art. 76 EPC:
**21847968.1 / 4 274 435**

(71) Applicant: **L'OREAL
75008 Paris (FR)**

(72) Inventors:
• **VEDEL, Charlotte
2100 Copenhagen Ø (DK)**
• **BILLEKOP OLSEN, Katja
2100 Copenhagen Ø (DK)**
• **TESDORPF, Jens Edward
2100 Copenhagen Ø (DK)**
• **KJÆRULFF, Søren
2100 Copenhagen Ø (DK)**

(74) Representative: **Larsen & Birkeholm A/S
Bredgade 3, 3rd floor
1260 Copenhagen K (DK)**

Remarks:
This application was filed on 17-04-2026 as a
divisional application to the application mentioned
under INID code 62.

## (54) STRAINS, COMPOSITIONS AND METHODS OF USE

(57) The present invention relates to a composition comprising *Lactiplantibacillus plantarum,* LB679R, deposited under the assession number DSM 33731.

**EP 4 768 035 A2**

**Description**

**Technical field of the invention**

The present invention relates to novel lactic acid bacterial strains, which alone or in combination can be used as probiotics. The invention also relates to pharmaceutical compositions, supplement compositions and products for personal care comprising the strains alone or in combination. In particular, the present invention relates to use of the strains for prevention or treatment of Candidiasis.

**Background of the invention**

The incidence of fungal infections has increased significantly in the last several decades, this fact is mainly due to the rise in antimicrobial resistance and the limited number of efficient antifungal drugs, which still have many side effects. Of the fungi considered as human pathogens, some members of the genus Candida are the most commonly recovered from fungal infections (Silva et al. 2012). The use of broad-spectrum antibiotics, denture stomatitis, catheters and parenteral nutrition, the presence of immunosuppression, the disruption of mucosal barriers, and the use of chemotherapy and radiotherapy as well as dys-functional microbiomes are among the most significant predisposing factors for invasive fungal infections development. The Candida genus comprises over 150 heterogeneous species, but only a minority are associated to human candidiasis, since roughly 65 % of Candida species are unable to grow at a temperature of 37 °C, enabling them to prosper as pathogens or commensals of humans. The genus is composed of a heterogeneous group of organisms, and more than 17 different Candida species are known to be etiological agents of human infections; however, more than 90 % of invasive infections are due to Candida species (Pfaller, MA. & DJ. Diekema 2007).

The pathogenicity of Candida species is attributed to certain virulence factors, such as the ability to evade host defenses, formation of hyphae, adhesion and biofilm formation (on host tissue or on medical devices), and the production of tissue-damaging hydrolytic enzymes such as proteases, phospholipases, and haemolysins (Silva et al 2012).

Biofilms are biological communities with an extraordinary degree of organization, in which microorganisms form structured, coordinated, and functional communities, embedded in a self-created extracellular matrix. Biofilm production is also associated with a high level of antimicrobial resistance of the associated organisms. The ability of Candida species to form drug-resistant biofilms is an important factor in their contribution to human disease, but it is also recognized that the more general provision of new medical practices (immunosuppressive therapy, invasive surgical procedures, the use of broad-spectrum antibiotics and diseases caused by dys-functional microbiomes) are highly significant as well.

Antifungal drugs are active by either killing the fungal cells, e.g., affecting a substance in the cell wall, causing a leak out of the cell contents and death, or by preventing fungal cells from growing and reproducing. There are many antifungal classes: polyenes, which includes amphotericin, nystatin, and pimaricin; azoles, including fluconazole, itraconazole, ketoconazole, miconazole, voriconazole, posaconazole, and rosaconazole; echinocandins, such as caspofungin and micafungin; and allylamines, including naftifine, terbinafine, morpholine drug, amorolfine, and griseofulvin; and the antimetabolite antifungal drugs, in which 5-fluorocytosine is incorporated. Common for these drugs are the increasing problems with development of resistance, lack of efficiency as well as the toxicity of the compounds.

Amphotericin B and fluconazole are among the antifungal agents most widely used to treat systemic fungal infections. The former has its use limited due to its high degree of toxicity in humans. The latter is frequently prescribed to treat Candida infections, although a great number of species of Candida display fluconazole resistance.

Candida species are capable of colonizing host tissues as well as abiotic surfaces, where it develops as a multilayered biofilm structure. An important factor correlated with the adhesion ability of Candida species is the presence of specific proteins on its cell wall, namely, adhesins. In C. glabrata, a major group of adhesins is encoded by the Epa (epithelial adhesin) gene family. The overall structure of Epa proteins is similar to that of the Als (agglutinin-like sequence) proteins of C. *albicans.* The ALS genes are one example of a gene family associated with pathogenicity mechanisms in C. *albicans* and other Candida species.

Biofilm forming ability may confer Candida species an ecological advantage, aiding survival as commensals and pathogens of humans by allowing them to evade host immune mechanisms, resist antifungal treatment, and withstand the competitive pressure from other microorganisms. Biofilm formation besides possibly being a key factor for the survival of these species, may also be responsible for Candida species being particularly well adapted to the colonization of tissues and indwelling medical devices.

The adherence of C. *albicans* to host cells or polymers is an essential and necessary first step in successful colonization and the development of pathogenesis and infection. *Candida albicans* is able to adhere directly or via an intermediary layer to e.g. acrylic resin dentures, which is the first step of the development of the infectious process. Several factors implicate in the adhesion of Candida to different surfaces including surface structure, properties and composition of biomaterials, hydrophobicity and roughness influence the adhesion of Candida.

In the Candida genus, the so-called virulence factors comprise not only the adhesion, hyphae formation and biofilm formation, but also the ability to destroy host tissues, which may be facilitated by the release of hydrolytic enzymes into the local environment. The general secretion of enzymes, such as proteases, phospholipases, lipases, and haemolysins, help the yeasts to survive and replicate within macrophages, being able to destroy the epithelium.

Phospholipases (PLs) hydrolyse phospholipids into fatty acids and their production contribute to host cell membrane damage, promoting cell damage or exposition of receptors that facilitate adherence. Candida are known to produce lipases and phospholipases, which clearly promotes a greater and powerful interface with the host mucosae, destroying it and endorsing an effective invasion of the tissues involved. Also, haemolysins are known to be putative virulence factors facilitating hyphal invasion in disseminated candidiasis. Recently, some authors reported that many Candida species, including *C. glabrata,* exhibit varying ability to produce alpha- or beta-haemolysins (Luo et al. 2001)

Candida species, including *C. glabrata* can grow in the host by degrading haemoglobin and extracting the elemental iron from host cells for metabolic processes (Negri M et al 2010). Haemolysins are known to be putative virulence factors backing pathogenicity in Candida species, the genetic expression of haemolytic activity in is poorly understood.

Antifungal resistance is both complex and multifaceted. It can be inducible in response to a compound or be an irreversible genetic change resulting from prolonged exposure. In detail, these include alterations or even an overexpression of target molecules, active extrusion through efflux pumps, limited diffusion, tolerance, and cell density, which are all characterized mechanisms utilized by fungi to combat the effects of antifungal treatments. Planktonic cells generally rely on irreversible genetic changes to maintain a resistant phenotype, whereas biofilm cells are able to persist due to their physical presence and the density of the population, which provides an almost inducible resistant phenotype irrespective of defined genetic alterations. Thus, Candida has an intrinsically reduced susceptibility to commonly used antifungal drugs due to different properties.

*Candida albicans* is a dimorphic commensal fungus that colonizes healthy human skin, mucosa, oral and the reproductive tract. *C. albicans* is also a predominantly opportunistic fungal pathogen, leading to disease manifestations such as disseminated candidiasis and chronic mucocutaneous candidiasis (CMC).

Candida species belong to the normal microbiota of the oral cavity, skin, gastrointestinal and vaginal tracts, and are responsible for several clinical manifestations, from mucocutaneous overgrowth to bloodstream infections. Once believed to be non-pathogenic, Candida spp. were rapidly blamable for many human diseases. Year after year, these pathological circumstances are more recurrent and problematic to treat, especially when patients reveal any level of immunosuppression or has a dys-functional microbiome e.g. with decreased microbial diversity. These difficulties arise from phenotypes of Candida being able to form biofilms and also from its high resistance to traditional antifungal therapies. Pathogenic fungi in the genus Candida can cause both superficial and serious systemic disease, and are now recognized as major agents of hospital-acquired infection. Many Candida infections involve the formation of biofilms on implanted devices such as indwelling catheters or prosthetic heart valves. Biofilms of *Candida albicans* formed in vitro on catheter material consist of matrix-enclosed microcolonies of yeasts and hyphae, arranged in a bilayer structure.

The biofilms are resistant to a range of antifungal agents currently in clinical use, including amphotericin B and fluconazole, and there appear to be multiple resistance mechanisms.

Lactic acid bacteria are a part of the microbial flora of the human gut, mouth, and vagina. Lactic acid bacteria play an important role in protecting the human body from infection via production of acids and acidification of the e.g. vagina, by production of other antimicrobial products, such as hydrogen peroxide $H_2O_2$, antimicrobial peptides or biosurfactants. Many topical, vaginal, oral and systemic medications may kill lactic acid bacteria. Hence, treatment of infections with antibiotics may place the body at increased risk for repeated acquisition of the infection or new infections caused by resistant microorganism e.g. Candidiasis.

For some decades, antifungal agents have been successfully used to prevent mucosal as well as invasive fungal infections. However, due to the drug side effects (nausea, vomiting, and diarrhea) and the emergence of resistant strains, antifungal prophylaxis has not been totally successful. The biofilm phenotype and phase of Candida is much more resistant to all these antifungals compared with their planktonic counterparts. The limited effect and the gradual emergence of resistance to antifungal drugs are a concern, thus alternative therapies are urgently warranted.

Use of probiotic microorganisms against candidiasis is an attractive alternative therapeutic to treat or prevent Candida infections in view of the limitation of the currently available antimicrobial compounds.

Lactic acid bacteria for intravaginal or oral use have been available for over 50 years in the form of probiotic preparations available in health food stores or as dairy products. These products have included vaginal suppositories containing lyophilized Lactobacillus spp. These products have been largely non-efficacious in treating infections due to the failure of the products to colonize the vagina or gastrointestinal tract with the exogenous Lactobacilli, prevent biofilm formation of pathogens or to a lack of antimicrobial activity in vivo. Typically the traditional probiotics are used to maintain a healthy gut flora and not known for any targeted or specific antimicrobial mechanisms.

Commercially available Lactobacillus has been shown to be unable to prevent biofilm formation of pathogens nor do the commercial Lactobacillus have antimicrobial activity against the persistent Candida infections. Most commercially available probiotic strains are not systematically selected for their antimicrobial activity but chosen due to their ability to be upscaled (Kwak, Y-K. et al. 2017).

The ability of Candida app. to form biofilms contributes to the high rates of recurrence that are typical for candidiasis and which unfortunately make repeated antifungal therapy inevitable. Hence lactic acid bacteria which are able to prevent, inhibit or reduce the development of biofilm by Candida spp. would be advantageous.

Further there is a need for lactic acid bacteria for the treatment of infections wherein the lactic acid bacteria maintain the antimicrobial activity in co-cultures, ability to acidify and prevent biofilm formation in the presence of Candida spp.

The present invention provides new probiotic strains and compositions which are targeted to at least one virulence mechanism of Candida, included is antimicrobial activity determined as growth inhibition, inhibition of biofilm attachment or formation or the ability to prevent Candida from their potency to secrete virulence factors as phospholipase, proteinase and haemolytic factor.

## Summary of the invention

Thus, an object of the present invention relates to new lactic acid bacteria as well as compositions, topical compositions, vaginal compositions, eye compositions, ear compositions or oral compositions comprising said new lactic acid bacteria, and their ability to prevent or treat candidiasis and Candida infections.

In particular, it is an object of the present invention to provide a new lactic acid bacterial strain and compositions comprising this new lactic acid bacterial strain that solves the above-mentioned problems of the prior art with biofilm formation, in particular biofilm formation of Candida pathogens, and thereby prevent infection.

Thus, one aspect of the invention relates to a composition comprising *Pediococcus pentosaceus* LB606R, deposited under the assession number DSM 33730; *Lactiplantibacillus plantarum,* LB679R, deposited under the assession number DSM 33731; or a combination hereof.

Another aspect of the present invention relates to an isolated bacterial strain selected from *Pediococcus pentosaceus LB606R,* deposited under the deposit assession number DSM 33730; and/or one or more bacteria strain selected from *Lactiplantibacillus plantarum* LB 679R, deposited under the deposit assession number DSM 33731.

Yet another aspect of the present invention relates to a composition comprising one or more bacterial strains selected from one or more *Pediococcus* strains; and/or one or more bacteria strains selected from one or more *Lactiplantibacillus* strains for use as a medicament.

Still another aspect of the present invention relates to a composition comprising one or more bacterial strains selected from one or more *Pediococcus* strains; and/or one or more bacteria strains selected from one or more *Lactiplantibacillus* strains for use in the prevention and/or treatment of candidiasis in a human or in an animal.

A further aspect of the present invention relates to a composition comprising one or more bacterial strains selected from one or more *Pediococcus* strains; and/or one or more bacteria strains selected from one or more *Lactiplantibacillus* strains for use in the prevention, inhibition, or treatment of biofilm formation.

An even further aspect of the present invention relates to a method for preventing biofilm formation in an environment, wherein the biofilm comprises candida species comprising the steps of: administering to the environment an effective amount of a lactic acid bacteria with antimicrobial activity, wherein the environment is a home, workplace, laboratory, industrial environment, hospital environment, aquatic environment, medical device, dental device, epithelial cells, mucous membranes, an animal or a human body.

A further aspect of the present invention relates to a food or feed ingredient, and a personal hygiene product, comprising the composition according to claims 1-6.

## Brief description of the figures

Figure 1 shows the Co-aggregation of C. albicans strain L26 and LB606R. The control of C. albicans L26 (Fig. 1a) shown with the combination and co-aggregation with LB606R (Fig. 1b), and

Figure 2 shows Growth inhibition by CFS of LB606R (Fig. 2a) and LB679R (Fig. 2b) against growth of C. albicans strain L26 at various concentrations of LB606R (Fig.2a) and LB679R (Fig. 2b).

The present invention will now be described in more detail in the following.

## Detailed description of the invention

Accordingly, the inventors of the present invention found particular lactic acid bacteria having antimicrobial activity targeted towards *Candida albicans.*

It is believed that the lactic acid bacteria of the present invention are able to colonize mucous membranes and/or prevent biofilm formation by pathogenic Candida spp. and/or have antimicrobial activity against pathogenic Candida spp. In particular *Candida albicans.*

The Lactic acid bacteria may be selected from a Pediococcus strain and/or a Lactiplantibacillus strain. preferably the lactic acid bacteria may be selected from the group consisting of *Pediococcus pentosaceus* LB606R deposited on 14th of December 2020 with DSMZ German Collection of Microorganisms and Cell Cultures GmbH as DSM 33730 and *Lactiplantibacillus plantarum* deposited as DSM 33731.

The Lactic acid bacteria selected from a Pediococcus strain (preferably the lactic acid bacteria selected from Pediococcus pentosaceus LB606R deposited on 14th of December 2020 with DSMZ German Collection of Microorganisms and Cell Cultures GmbH as DSM 33730.

In an embodiment of the present invention the composition may be a pharmaceutical composition comprising one or more lactic acid bacteria according to the present invention together with a pharmaceutically acceptable carrier and/or diluent.

Preferably, the pharmaceutical composition comprising $10^3$ to $10^{13}$ colony forming units of lactic acid bacteria according to the present invention per gram. More specifically a pharmaceutical composition comprising $10^6$ to $10^{12}$ colony forming units of lactic acid bacteria per gram. More specifically a pharmaceutical composition comprising $10^7$ to $10^{11}$ colony forming units of lactic acid bacteria per gram.

The pharmaceutical composition may be in the form of a suspension, spray, gel, cream, lotion, powder, capsule, oil, solution for lavages, ovules, a vaginal insert, a suppository, lozenge, tablets, microencapsulated product or in form of a food supplement or a food product.

The composition may be provided with a therapeutic effective amount of the lactic acid bacteria, in particular:

- a therapeutic effective amount of the one or more bacteria strain selected from a *Pediococcus* strain, most preferably a therapeutic effective amount of *Pediococcus pentosaceus LB606R,* deposited under the deposit assession number DSM 33730; or
- a therapeutic effective amount of the one or more bacteria strain selected from a *Lactiplantibacillus* strain, most preferably a therapeutic effective amount *Lactiplantibacillus plantarum* LB 679R, deposited under the deposit assession number DSM 33731; or
- a combination hereof

In the context of the present invention, the *Pediococcus* strain, such as the of *Pediococcus pentosaceus LB606R,* deposited under the deposit assession number DSM 33730 may be more preferred compared to the *Lactiplantibacillus* strain, such as the *Lactiplantibacillus plantarum* LB 679R, deposited under the deposit assession number DSM 33731

In the context of the present invention the terms "therapeutic effective amount" or "effective amount" may refer to an amount of the compounds in a composition or preparation which, when administered as part of a desired dosage regimen (to a human or an animal, preferably a human) alleviates a symptom, ameliorates a condition, or slows the onset of disease conditions according to clinically acceptable standards for the disorder or condition to be treated or the cosmetic purpose, e.g., at a reasonable benefit risk ratio applicable to any medical treatment.

For the practical use, the microorganisms of the invention may be formulated in suitable administration forms such as gels, creams, capsules, tablets, solutions for lavages, sachets, any food products and the like. Unit doses may comprise from 10 to $10^{13}$ cells of each single strain, the preferred dosage being above $10^5$ cells per unit dose. More preferable dosage is above $10^6$ cells per unit dose.

The bacterial cultures may be stabilized in freeze dried, lyophilized or microencapsulated forms and may be prepared according to conventional methods.

For the preparation of aqueous formulations for lavages and irrigations a two-phase system can be used. E.g. small bottles with reservoirs containing the lyophilized microorganisms, to be dissolved before use in a suitable liquid carrier contained in the bottles.

For the preparation of creams and gels, the lactic acid bacteria are stabilized in the formulation. Preferable in formulations with low water activity or in a microencapsulated form.

In an embodiment of the present invention the one or more lactic acid bacteria strain according to the present invention may be provided in a pharmaceutical composition for preventing and/or treating vaginal infections.

In yet an embodiment of the present invention the one or more lactic acid bacteria strain according to the present invention may be provided in a pharmaceutical composition for preventing and/or treating urinary-tract infections.

In a further embodiment of the present invention the one or more lactic acid bacteria strain according to the present invention may be provided in a pharmaceutical composition for preventing and/or treating topical, skin, eye, ear or mucosal infections.

In still a further embodiment of the present invention the one or more lactic acid bacteria strain according to the present invention may be provided in a pharmaceutical composition for preventing and/or treating oral infections.

A further embodiment of the present invention the composition and/or the one or more lactic acid bacteria according to the present invention are used in combination with a medical device.

Yet an embodiment of the present invention relates to a method for treating or preventing microbial imbalances in mucous membranes in a human or in an animal comprising administration of an effective amount of lactic acid bacteria according to the invention.

In the context of the present invention the term "prevent" may be art-recognized, and when used in relation to a condition, such as a local recurrence, may be well understood in the art, and includes administration of a composition which reduces the frequency of, or delays the onset of, symptoms of a medical condition in a subject relative to a subject which does not receive the composition. Thus, prevention of infection includes, for example, reducing the number of detectable pathogenic microorganisms in a population of patients receiving a prophylactic treatment relative to an untreated control population, and/or delaying the appearance of detectable lesions in a treated population versus an untreated control population, e.g., by a statistically and/or clinically significant amount.

As used herein, the terms "treating" or 'treatment" includes reversing, reducing, or arresting the symptoms, clinical signs, and underlying pathology of a condition in a manner to improve or stabilize a subject's condition.

As used herein, and as well-understood in the art, "treatment" may be an approach for obtaining beneficial or desired results, including clinical results. For purposes of this subject matter, beneficial or desired clinical results include, but are not limited to, alleviation or amelioration of one or more symptoms, diminishment of extent of disease, stabilized (i.e., not worsening) state of disease, prevention of disease, delay or slowing of disease progression, and/or amelioration or palliation of the disease state.

The decrease can be at least a 10% decrease in severity of complications or symptoms, such as at least 20% decrease, e.g. at least 30% decrease, such as at least 40% decrease, e.g. at least 50% decrease, such as at least 60% decrease, e.g. at least 70% decrease, such as at least 80% decrease, e.g. at least 90% decrease, such as at least 95% decrease, e.g. at least 98% decrease, such as at least 99% decrease, e.g. 100% decrease in severity of complications or symptoms.

The term "lactic acid bacteria" includes species from the families Lactobacillaceae, Aerococcaceae, Bifidobacteriaceae, Carnobacteriaceae, Enterococcaceae, Leuconostocaceae and Streptococcaceae. These are considered non-pathogenic and are used as probiotic bacteria in general to improve gastrointestinal flora and in the treatment of gastrointestinal symptoms. Lactic acid bacteria according to the present invention are preferably selected from a lactic acid bacterial strain inhibiting, preventing or reducing biofilm formation. In particular, biofilm formation of one or more yeasts. More specifically, biofilm formation of pathogenic *Candida* species. Even more specifically, biofilm formation of *Candida albicans.*

The inventors of the present invention surprisingly found certain strains of lactic acid bacteria showed to be able to inhibiting, preventing or reducing biofilm formation.

Thus, a preferred aspect of the present invention relates to a composition comprising *Pediococcus pentosaceus* LB606R, deposited under the assession number DSM 33730; *Lactiplantibacillus plantarum,* LB679R, deposited under the assession number DSM 33731; or a combination hereof.

Preferably, the composition according to the present invention comprises *Pediococcus pentosaceus* LB606R, deposited under the assession number DSM 33730.

The composition according to the present invention may comprise viable or non-viable cells of lactic acid bacteria according to the present invention.

In an embodiment of the present invention the one or more *Pediococcus* strains (such as *Pediococcus pentosaceus LB606R,* deposited under the deposit assession number DSM 33730); and/or the one or more *Lactiplantibacillus* strains, (such as *Lactiplantibacillus plantarum* LB 679R, deposited under the deposit assession number DSM 33731) may be provided as viable cells, as non-viable cells, as a lysate, as a fraction, as a ferment, as metabolites, as an extract, as a derivative, as analogues, or as a mutant of one of the *Pediococcus* strains or the *Lactiplantibacillus* strains according to the present invention.

Preferably, the one or more *Pediococcus* strains (such as *Pediococcus pentosaceus LB606R,* deposited under the deposit assession number DSM 33730); and/or the one or more *Lactiplantibacillus* strains, (such as *Lactiplantibacillus plantarum* LB 679R, deposited under the deposit assession number DSM 33731) may be provided as viable cells, as non-viable cells, or as a lysate.

An embodiment of the present invention relates to a composition comprising viable/live probiotic strains according to the present invention for use in treating; alleviating, suppressing; prophylaxis; and/or preventing growth of a pathogenic micro-organism.

More preferably the present invention may provide a composition as defined herein for use in the treatment, alleviating, suppressing; prophylaxis of one or more pathogenic bacterial infection in a human or an animal.

In an embodiment of the present invention the animal may include, but are not limited to, primates, farm animals, sport animals, rodents and pets. More specifically the animals may include mice, rats, hamsters, and guinea pigs; rabbits; dogs; cats; sheep; pigs; piglets; sows; poultry; turkeys; broilers; minks; goats; cattle; horses; and non-human primates such as apes and monkeys.

More preferably the present invention may provide a composition as defined herein for use in preventing growth of a pathogenic micro-organism.

A "decrease" in growth may be "statistically significant" as compared to the growth period in the absence of the bacterial strains of the present invention, and may include a 10 percent, 20 percent, 30 percent, 40 percent, 50 percent, 60 percent, 70 percent, 80 percent, 85 percent, 90 percent, 95 percent, 99 percent or 100 percent decrease.

In an embodiment of the present invention the growth inhibition may be determined as a decrease in growth of at least 25 percent. Preferably the growth inhibition is determined as a decrease in growth of at least 50 percent, more preferably the growth inhibition is determined as a decrease in growth of at least 80 percent more preferably the growth inhibition is determined as a decrease in growth of at least 90 percent.

"Probiotics" may be defined as live microorganisms that, when administrated or consumed in adequate quantities, confer health benefits on the host.

The non-viable cells according to the present invention may be provided as a lysate, as a fraction, as a ferment, as metabolites, as an extract, as a derivative, as analogues, or as a mutant of one of the *Pediococcus* strains or the *Lactiplantibacillus* strains according to the present invention

"Lysates", "derivatives", "analogues", "fractions" or "extracts" may be obtained from dead or killed lactic acid bacteria. These lysates, fractions, derivative, analogues, and extracts preferably have the properties of being able to bind or co-aggregate with pathogenic bacteria thereby preventing growth and/or biofilm formation of a pathogen, where "lysate" as well as the term "extract" refers in particular to a solution or suspension in an aqueous medium of the cells of the microorganism and/or metabolites according to the invention and comprises, for example, macromolecules such as DNA, RNA, proteins, peptides, lipids, carbohydrates, etc. as well as cell detritus. The lysate preferably includes the cell wall or cell wall constituents including binding receptors for co-aggregation. Methods of producing lysates are sufficiently well known to those skilled in the art and includes, for example, the use of a French press or enzymatic lysis, a ball mill with glass beads or iron beads. Cells can be broken open by enzymatic, physical or chemical methods. Examples of enzymatic cell lysis may include individual enzymes as well as enzyme cocktails, for example, proteases, proteinase K, lipases, glycosidases; chemical lysis may be induced by ionophores, detergents such as SDS, acids or bases; physical methods may also be implemented by using high pressures such as the French press, osmolarities, temperatures or alternating between heat and cold. Furthermore chemical, physical and enzymatic methods may of course be combined. "Extract" can be any of these cellular components, metabolites, ferment, or cell fractions.

A mutant of one of the *Pediococcus* strains or the *Lactiplantibacillus* strains according to the present invention may be provided by a method to obtain a mutant of one of the following strains; *Pediococcus pentosaceus* LB606 deposited with Germena Collection of Microorganisms and Cell Cultures under the under the accession number DSM 33730 or *Lactiplantibacillus plantarum* LB679R deposited as DSM 33731, comprising using the deposited strain as starting material and applying mutagenesis, wherein the obtained mutant retains or enhances the probiotic and/or antimycotic and/or antimicrobial properties and/or the capacity to inhibit candidiasis.

In an embodiment of the present invention the composition may be free from, or substantially free from, viable microorganisms. The composition can comprise cell material including dead cells in form of a lysate or a ferment. In a further embodiment of the present invention the composition may comprise the supernatant from fermentation and the cell material having a further functional effect.

In an embodiment of the present invention the lactic acid bacteria according to the present invention may be capable of co-aggregating with *Candida* strains or inhibit *Candida* strains.

The microorganisms according to the invention are preferably in isolated or purified form, where the term "isolated" means in particular that the lactic acid bacteria are derived from their culture medium including their natural medium.

The term "inhibition" or "inhibit" as used herein, means the killing of a microorganism, such as an undesired microorganism, or the control of the growth of said microorganism. Including inhibition of biofilm formation which can be inhibition of the initial attachment of a microorganism or inhibition of the further formation of a biofilm by growth inhibition of the biofilm forming microorganism.

In an embodiment of the present invention the composition and/or the lactic acid bacteria according to the present invention may inhibit biofilm formation.

The lactic acid bacteria may preferably be one or more *Pediococcus* strains, such as *Pediococcus pentosaceus LB606R,* deposited under the deposit assession number DSM 33730; and/or one or more *Lactiplantibacillus* strains, such as *Lactiplantibacillus plantarum* LB 679R, deposited under the deposit assession number DSM 33731.

A preferred embodiment of the present invention relates to an isolated bacterial strain selected from *Pediococcus pentosaceus LB606R,* deposited under the deposit assession number DSM 33730; and/or one or more bacteria strain selected from *Lactiplantibacillus plantarum* LB 679R, deposited under the deposit assession number DSM 33731.

An embodiment of the present invention relates to an isolated bacterial strain selected from *Pediococcus pentosaceus LB606R,* deposited under the deposit assession number DSM 33730; and/or one or more bacteria strain selected from *Lactiplantibacillus plantarum* LB 679R, deposited under the deposit assession number DSM 33731, wherein said strains presents antimicrobial activity.

The microorganisms according to the present invention may preferably be in isolated form, where the term "isolated" means in particular that the lactic acid bacteria are derived from their culture medium including their natural medium, for example isolated from other species.

The antimicrobial activity may be normalizing a dysfunctional vaginal microbiota and/or inhibition of biofilm formation. In particular, the isolated bacterial strain selected from *Pediococcus pentosaceus LB606R,* deposited under the deposit assession number DSM 33730; and/or one or more bacteria strain selected from *Lactiplantibacillus plantarum* LB 679R, deposited under the deposit assession number DSM 33731 may inhibit biofilm formation of one or more yeasts. More specifically, the isolates strains may inhibit biofilm formation of pathogenic *Candida* species. Even more specifically, the isolates strains may inhibit biofilm formation of *Candida albicans.*

A "dysfunctional microbiota" or "dysfunctional vaginal microbiota" can be determined by either the Nugent score or a diagnosis done by evaluating the pH, the presences of *Lactobacillus spp.* versus a mixed flora consisting of *Gardnerella vaginalis,* Bacteroides species or *Candida* species or by use of the Amsel Criteria for bacterial vaginosis which includes pH, evaluating the presence of clue cells, white discharge and an odour of amines after mixing with KOH.

The antimicrobial activity may be treatment of candidiasis in a human or in an animal; or inhibition of biofilm formation on a surface as defined herein.

In an embodiment of the present invention, the composition inhibits biofilm formation. In particular, the composition of the present invention inhibits biofilm formation of one or more yeasts. More specifically, the composition of the present invention inhibits biofilm formation of pathogenic *Candida* species. Even more specifically, the composition of the present invention inhibits biofilm formation of *Candida albicans.*

In the present context the inhibition of biofilm formation may be an at least 25% biofilm inhibition, such as an at least 50% biofilm inhibition, e.g. an at least 75% biofilm inhibition, such as an at least 85% biofilm inhibition, e.g. an at least 90% biofilm inhibition, such as an at least 95% biofilm inhibition, e.g. an at least 98% biofilm inhibition.

The bactaria *Lactiplantibacillus plantarum,* was previously named *Lactobacillus plantarum,* and may be a member of the genus *Lactiplantibacillus* and commonly found in many fermented food products as well as anaerobic plant matter.

The inventors of the present invention identified and isolated the specific strains of *Pediococcus pentosaceus* (LB606R/DSM 33730) and *Lactiplantibacillus plantarum* (LB679R/DSM 33731) and surprisingly identified one or more specific activities provided by the specific strains, like inhibition of biofilm formation, and/or for the treatment of dysfunctional vaginal microbiota.

In an embodiment of the present invention, the composition according to the present invention may be used for inhibiting biofilm formation on a surface. Preferably, the surface may be a surface in a hospital, and/or a surface of a medical device.

In yet an embodiment of the present invention, the composition according to the present invention may be used for inhibiting biofilm formation in or on the human or animal body.

In the present context, biofilm formation may be produced by one or more microorganism. In particular, the one or more microorganism may be a yeast. The one or more yeast may be one or more pathogenic *Candida* species. The one or more pathogenic *Candida* species may be one or more *Candida albicans.*

In an embodiment of the present invention the composition (or the lactic acid bacteria) according to the present invention may be formulated or used in a medicament, a pharmaceutical, a food product, a feed product, a disinfectant, or in a personal care product.

The disinfectant according to the present invention may be used for treating a surface to avoid biofilm formation on said surface. The surface may be a medical or a non-medical surface. The medical surface may be a surface in a hospital or the surface of a medical device. The medical device may include a medical device to be implanted into, or used inside, a human or an animal or a medical device mainly used outside the human or animal body.

The composition according to the present invention, and/or the one or more of the isolated bacterial strains of the present invention may be used in the treatment of one or more of the infections which has arisen from: surgical wounds, decubitus ulcers, infections from catheters, stents, cardiocirculatory devices, prostheses, prosthetic insertions, otologic, orthopaedic and dental prostheses, screws and nails, oral cavity infections and infections of the oral and vaginal mucosa, local infections, otitis, rhinosinusitis, pharyngitis, laryngitis and pneumonia.

In an embodiment of the present invention the composition according to the present invention and/or the one or more of the isolated bacterial strains of the present invention may be effective against antibiotic resistant microorganisms. In particular, effective against an antibiotic resistant infection.

The use of the composition (or the lactic acid bacteria) according to the present invention as a disinfectant may reduce the risk of yeast infection, in particular reduce the risk of infection by pathogenic *Candida,* even more particularly, reduce the risk of infection by *Candida albicans.*

An embodiment of the present invention relates to one or more bacterial strains selected from one or more *Pediococcus* strains; and/or one or more bacteria strains selected from one or more *Lactiplantibacillus* strains for use as a medicament.

In an embodiment of the present invention the composition consists essentially of one or more bacterial strains selected from one or more *Pediococcus* strains; and/or one or more bacteria strains selected from one or more *Lactiplantibacillus* strains for use as a medicament.

In the context of the present invention, the term "consisting essentially of", relates to a limitation of the scope of a claim to the specified features or steps and those features or steps, not mentioned and that do not materially affect the basic and novel characteristic(s) of the claimed invention.

A preferred embodiment of the present invention relates to a composition comprising one or more bacterial strains selected from one or more *Pediococcus* strains; and/or one or more bacteria strains selected from one or more *Lactiplantibacillus* strains for use in the prevention and/or treatment of candidiasis in a human or in an animal.

Preferably, the prevention and/or treatment of candidiasis in a human or in an animal may be vaginal prevention and/or treatment of candidiasis; oral prevention and/or treatment of candidiasis; and/or intestinal prevention and/or treatment of candidiasis.

The present invention may relate to a composition consisting essentially of one or more bacterial strains selected from one or more *Pediococcus* strains; and/or one or more bacteria strains selected from one or more *Lactiplantibacillus* strains for use in the prevention and/or treatment of candidiasis in a human or in an animal.

A preferred embodiment of the present invention relates to a composition comprising one or more bacterial strains selected from one or more *Pediococcus* strains; and/or one or more bacteria strains selected from one or more *Lactiplantibacillus* strains for use in the prevention, inhibition, or treatment of biofilm formation.

In an embodiment of the present invention the biofilm formation may be a yeast biofilm formation, such as a pathogenic *Candida* species biofilm formation; in particular a *Candida albicans* biofilm formation.

In a further embodiment of the present invention the composition consists essentially of one or more bacterial strains selected from one or more *Pediococcus* strains; and/or one or more bacteria strains selected from one or more *Lactiplantibacillus* strains for use in the prevention and/or treatment of candidiasis in a human or in an animal.

In an embodiment of the present invention the composition according to the present invention may decrease the virulence of yeasts; such as pathogenic *Candida* species; in particular *Candida albicans.*

In the context of the present invention the term "virulence" relates to the quality or property of the yeast/the pathogenic *Candida* (in particular *Candida albicans*) of being poisonous, venomous or injurious to life or wellbeing of a human or an animal.

The composition according to the present invention may preferably consist essentially of one or more bacteria strain selected from a *Pediococcus* strain.

The one or more bacteria strain selected from *Pediococcus* may preferably comprise *Pediococcus pentosaceus LB606R,* deposited under the deposit assession number DSM 33730.

The composition according to the present invention may consist essentially of one or more bacteria strain selected from a *Lactiplantibacillus* strain.

The one or more bacteria strain selected from *Lactiplantibacillus* may preferably comprise *Lactiplantibacillus plantarum* LB 679R, deposited under the deposit assession number DSM 33731.

The composition according to the present invention may consist essentially of one or more bacteria strain selected from a *Pediococcus* strain and a *Lactiplantibacillus* strain.

The one or more bacteria strain selected from *Pediococcus* may preferably comprise *Pediococcus pentosaceus LB606R,* deposited under the deposit assession number DSM 33730 and the one or more bacteria strain selected from *Lactiplantibacillus* may preferably comprise *Lactiplantibacillus plantarum* LB 679R, deposited under the deposit assession number DSM 33731.

A preferred embodiment of the present invention relates to a method for preventing biofilm formation in an environment, wherein the biofilm comprises candida species comprising the steps of: administering to the environment an effective amount of a lactic acid bacteria with antimicrobial activity, wherein the environment is a home, workplace, laboratory, industrial environment, aquatic environment, medical device, dental device, epithelial cells, mucous membranes, human or animal body.

The composition may be formulated for oral use, topical use, intestinal use or genital use.

Preferably, the composition according to the present invention is formulated for oral or genital use. Even more preferably, the composition according to the present invention is formulated for genital use.

In an embodiment of the present invention the genital use may be female genital use, such as vaginal use, and/or male genital use. Preferably, the genital use may be vaginal use.

During topical use of the composition of the present invention the composition may be used on the skin, on the mucosa, or on the nails of a human or animal.

In an embodiment of the present invention the composition may be formulated as a suspension, spray, gel, cream, lotion, powder, capsule, oil, solution for lavages, ovules, a vaginal insert, a suppository, lozenge, tablets, microencapsulated product or in form of a food supplement or a food product

The lactic acid bacteria may comprise one or more *Pediococcus* strains; one or more bacteria strains selected from one or more *Lactiplantibacillus* strains; or a combination hereof. The one or more *Pediococcus* strains may preferably comprise *Pediococcus pentosaceus LB606R,* deposited under the deposit assession number DSM 33730. The one or more *Lactiplantibacillus* strains may preferably comprise *Lactiplantibacillus plantarum* LB 679R, deposited under the deposit assession number DSM 33731.

A preferred embodiment of the present invention relates to a food or feed ingredient, or a food or feed product, comprising the composition according to the present invention.

A preferred embodiment of the present invention relates to a personal hygiene product comprising the composition according to the present invention.

The composition according to the present invention may be supplemented with further components to improve the effect of the composition, or to stabilize the composition, or both.

In an embodiment of the present invention the composition further comprises an antimycotic drug. This additional antimycotic drug may further improve the activity of the composition in preventing or inhibiting biofilm formation.

An aspect of the present invention relates to a medical device comprising the composition according to the present invention, or a vaginal microbiome transplant according to the present invention, or an isolated bacterial strain according to the present invention.

The composition according to the present invention may be formulated into medical device. Thus, the composition according to the present invention comprising one or more lactic acid bacteria according to the invention, may be used as a medical device.

The medical device according to the present invention may be formulated into a suspension, spray, gel, cream, lotion, powder, capsule, ointment, oil, solution for lavages, ovules, a vaginal insert, a suppository, lozenge, tablets, microencapsulated product.

In a further embodiment of the present invention the composition further comprises one or more prebiotic. The prebiotic may improve viability of the added lactic acid bacteria and/or the naturally present microbiota.

In an embodiment of the present invention the composition comprising the lactic acid bacteria may further comprise a prebiotic.

Prebiotics are non-digestible food components that increase the growth of specific microorganisms. "Synbiotics" are compositions comprising at least one probiotic and at least one prebiotic. Such compositions are understood to encourage the growth of beneficial bacteria (e.g. the probiotic). Thus, powerful synbiotics are based on a combination of specific strains of probiotic bacteria with carefully selected prebiotics. They can lead to an important health benefit to a human or in an animal.

An embodiment of the present invention the composition according to the present invention may comprise a symbiotic composition.

According to another embodiment of the present invention there may be provided a probiotic composition comprising the probiotic microorganism and at least one more active ingredient.

Prebiotics refer to chemical products that induce the growth and/or activity of commensal microorganisms (e.g., bacteria and fungi) that contribute to the well-being of their host. Prebiotics are nondigestible carbohydrates that are undigested or partly un-digested by the host and stimulate the growth and/or activity of advantageous bacteria that colonize the host.

Some oligosaccharides that may be used as prebiotics are fructo-oligosaccharides (FOS), xylooligosaccharides (XOS), polydextrose, pectins, galacto-oligo saccharides (GOS) or human milk oligosaccharides (HMO). Moreover, disaccharides like lactulose or lactose some monosaccharides such as tagatose can also be used as prebiotics.

The other active ingredient (or other ingredients) may not be limited in any way.

Prebiotics may be compounds which can be metabolized by probiotics. Preferably prebiotics are non-digestible or poorly digestible by a mammal. Prebiotics are well known in the art and when used in the present invention there is no particular limitation of the prebiotic as such.

In an embodiment of the present invention, at least one prebiotic may be added to the composition. Preferably, the at least one prebiotic may be selected from the following compounds and compositions: non-digestible carbohydrates, beta-glucans, mannan-oligosaccharides, inulin, oligofructose, human milk oligosaccharides (HMO), galactooligosaccharides (GOS), lactulose, lactosucrose, galactotriose, fructo-oligosaccaride (FOS), cellobiose, cellodextrins, cylodextrins, maltitol, lactitol, glycosilsucrose. Optionally, mannan-oligosaccharides and/or inulin may be preferred.

HMOs may include lacto-N-tetraose, lacto-N-fucopentaose, lacto-N-triose, 3'-sialyllactose, lacto-N-neofucopentaose, sialic acid, L-fucose, 2-fucosyllactose, 6'-sialyllactose, lacto-N-neotetraose and 3-fucosyllactose.

D- and L-fucose are considered to strengthen natural defence of skin, stimulate epidermis immune defence and/or prevent and/or treat cutaneous autoimmune disease. Thus, in an embodiment of the present invention the composition may comprises D- and/or L-fucose.

In yet an embodiment of the present invention the composition comprises L-fucose in a concentration in the range of 1 mM to 1000 mM, such as in the range of 10 mM to 500 mM, e.g. in the range of 25 mM to 250 mM.

The composition according to the present invention may further comprise at least one active ingredient.

In an embodiment of the present invention the composition may comprise at least one further probiotic microorganism selected from the group consisting of bacteria, yeasts or molds.

The at least one further probiotic microorganism may be selected from, but not restricted to, *Bifidobacterium lactis* DSM10140, *B. lactis* LKM512, *B. lactis* DSM 20451, *Bifidobacterium bifidum* BB-225, *Bifidobacterium adolescentis* BB-102, *Bifidobacterium breve* BB-308, *Bifidobacterium longum* BB-536 from Zaidanhojin Nihon Bifizusukin Senta (Japan Bifidus Bacteria Center), *Bifidobacterium* NCIMB 41675 described in EP2823822. *Bifidobacterium bifidum* BB-225, *Bifidobacterium adolescentis* BB-102, *Bifidobacterium breve* BB-308, *Bifidobacterium lactis* HN019 (Howaru), *Bifidobacterium bifidum* Bb-02, *Bifidobacterium bifidum* Bb-06, *Bifidobacterium longum* KC-1, *Bifidobacterium longum* 913, *B. lactis* BI-04, *B. lactis* Bi-07 available from DuPont Nutrition Biosciences ApS, *Bifidobacterium lactis* DN 173010 available from Groupe Danone, *Bifidobacterium lactis* Bb-12 available from Chr. Hansen A/S, *Bifidobacterium breve* M-16V (Morinaga) and/or a Lactobacillus having a probiotic effect and may be any of the following strains; *Lactobacillus rhamnosus* LGG (Chr. Hansen), *Lactobacillus gasseri* LN40, *L. gasseri* EB01TM, *L. rhamnosus* PB01TM *L. rhamnosus* LN113, *L. fermentum* LN99, *Lactobacillus acidophilus* NCFM, *Lactobacillus bulgaricus* 1260, *Lactobacillus paracasei* Lpc-37, *Lactobacillus rhamnosus* HN001 available from DuPont Nutrition Biosciences ApS, *Streptococcus thermophilus* 715 and *Streptococcus thermophilus* ST21 available from DuPont Nutrition Biosciences ApS, *Lactobacillus paracasei* subsp. *paracasei* CRL431 (ATCC *55544), Lactobacillus paracasei* strain F-19 from Medipharm, Inc. *L. paracasei* LAFTI L26 (DSM Food Specialties, the Netherlands) and *L. paracasei* CRL 431 (Chr. Hansen), *Lactobacillus acidophilus* PTA-4797, *L. salivarius* Ls-33 and *L. curvatus* 853 (DuPont Nutrition Biosciences ApS), *Lactobacillus pentosus* CECT 7504, *Lactobacillus plantarum* 299v (Probi AB), *Lactobacillus plantarum* LMC1 (DSM 32252), *Lactobacillus paracasei* LMC4 (DSM 32254), *Lactobacillus rhamnosus* LMC6 (DSM 32256), *Lactobacillus rhamnosus* LMC7 (DSM 32257), *Lactobacillus paracasei* LMC8 (DSM 32258), *Lactobacillus casei* ssp. rhamnosus LC705 is described in FI Patent 92498, Valio Oy, *Lactobacillus rhamnosus* LC705 (DSM 7061), *Propionic acid bacterium* eg. *Propionibacterium freudenreichii* ssp. *shermanii* PJS (DSM 7067) described in greater details in FI Patent 92498, Valio Oy, Nitrosomonas eutropha D23 (ABIome), *Staphylococcus hominis* strains A9, C2, AMT2, AMT3, AMT4-C2, AMT4-GI, and/or AMT4-D12. (all from Matrisys Bioscience), *Staphylococcus epidermidis* strains M034, M038, All, AMT1, AMT5-C5, and/or AMT5-G6 (all from Matrisys Bioscience), *L. plantarum* YUN-V2.0 (BCCM LMG P-29456), *L. pentosus* YUN-V1.0 (BCCN LMG P-29455), *L. rhamnosus* YUN-S1.0 (BCCM LMG P-2961) and/or any combinations hereof.

Additionally the at least one further probiotic microorganism may be selected from the group consisting of *Weissella viridescens* LB10G (DSM 32906), *Lactobacillus paracasei* LB113R (DSM 32907), *Lactobacillus plantarum* LB244R (DSM 32996), *Lactobacillus paracasei* LB116R (DSM 32908), *Lactobacillus brevis* LB152G (DSM 32995), *Lactobacillus paracasei* LB28R (DSM 32994), *Enterococcus faecium* LB276R (DSM 32997), *Leuconostoc mesenteriodes* LB349R (DSM 33093), *Lactobacillus plantarum* LB316R (DSM 33091), *Lactobacillus plantarum* LB356R (DSM 33094), *Lactobacillus plantarum* LB312R (DSM 33098); and/or any combinations hereof or mutant strains thereof and/or the cell lysate and/or the soluble metabolite of anyone of these probiotic strains.

In an embodiment of the present invention the probiotic strain may be used as a live isolated microorganism in a stabilized form. Suitable methods for stabilization are known to those skilled in the art and includes freeze drying, spray drying or lyophilization involving different cryoprotectants.

In a further embodiment of the present invention the strain may be used as a live isolated strain.

Preferably, the strain may be used as a live isolated stabilized strain. Even more preferably, the strain may be used as a live isolated strain stabilized by lyophilization. Even more preferably, the strain may be used as a live isolated strain stabilized by lyophilization and comprising a cryoprotectant and incorporated into a formulation suitable for oral, vaginal or topical use.

The composition according to the present invention may comprise at least one bacterial strain in combination with at least one further probiotic microorganism, wherein the at least one further probiotic microorganism may be selected from but not restricted to a strain selected from the genus Lactobacillus.

Deposit of biological material

The lactic acid bacteria according to the present invention include in particular microorganisms or analogs, fragments, derivatives, fermentates, lysates, mutants or combinations obtained from the microorganism, where the microorganism is deposited on 14th of December 2020 with the German Collection for Microorganisms and Cell Cultures: *Pediococcus pentosaceus* LB606R deposited as DSM 33730 and *Lactiplantibacillus plantarum* deposited as DSM 33731.

It should be noted that embodiments and features described in the context of one of the aspects of the present invention also apply to the other aspects of the invention.

All patent and non-patent references cited in the present application, are hereby incorporated by reference in their entirety.

The invention will now be described in further details in the following non-limiting examples.

**Examples**

Example 1 Strain screening identification

Samples

For identification and selection of microorganisms according to the invention, a strain collection of new isolated lactic acid bacteria (LAB) was established. Samples from different origins, such as homemade sauerkraut, kimchi and healthy human donor samples (vaginal, oral, anal, skin, human milk, baby diapers) were collected for isolation of at least 1200 new lactic acid bacteria strains. The samples were collected on Man Rogosa Sharp (MRS, Sigma-Aldrich) broth and agar cultured anaerobically at 37°C overnight or until colony formation. The isolates are plated and sub-cultured until isolated colonies were obtained. The isolated colonies are stored in MRS broth with 25 % glycerol at -80°C for future use. Strains were identified using 16S rRNA Sanger sequencing standard methods.

Example 2 spot on agar assay

The +1200 LAB isolated (example 1) was screened for the ability to growth inhibit Candida albicans, a pathogen being associated with Candidiasis.
Candida strains were obtained from BEI resources:

Candida albicans, strain P57055, catalogue no. 29439 (blood isolate)
Candida albicans, strain L26, catalogue no. 29445 (vaginal isolate)
Candida albicans, strain 19F, catalogue no. 29449 (vaginal isolate)

Competition between LAB and Candida strains were determined according to the methods described in the following publications: Dowarah, R., et al. 2018, Selection and characterization of probiotic lactic acid bacteria and its impact on growth, nutrient digestibility, health and antioxidant status in weaned piglets. PLoS ONE, 13(3), Khare, A., & Tavazoie, S. (2015). Multifactorial Competition and Resistance in a Two-Species Bacterial System. PLoS Genetics, 11(12), 1-21.
Lactic acid bacteria (LAB) strains were incubated in Man Rogosa Sharp (MRS, Sigma-Aldrich) broth overnight at 37 °C. C. albicans was incubated overnight at 37 °C in YPD broth (20 g/L peptone, 10 g/L Yeast extract, 20 g/L glucose). The C. albicans culture with an OD600 of approximately 0.5 was diluted to $10^{-1}$ or $10^{-2}$ and 1 mL was plated on Mueller-Hinton agar plates to create a lawn. From LAB cultures, 20 $\mu$L were spotted on top of the Mueller-Hinton plates. Inhibition zones were measured after overnight incubation at 37 °C at aerobic conditions.

Example 3 Co-aggregation

Co-aggregation was determined according to known methods Cisar, J. O. et al. (1979). "Specificity of Coaggregation Reactions between Human Oral Streptococci and Strains of Actinomyces Viscosus or Actinomyces Naeslundii." Infection and Immunity 24 (3): 742-52. LAB and C. albicans were grown overnight at 37 °C. MRS broth was used for LAB and YPD broth was used for C. albicans. The cultures were harvested by centrifugation at 6000 rpm for two minutes. The supernatants were discharged, and the pellet was washed once in 500 $\mu$L phosphate buffered saline (PBS) and then resuspended in 1 mL PBS. From the C. albicans culture and each of the LAB cultures, 0.5, 0.25, $10^{-1}$, $10^{-2}$, and $10^{-3}$ dilutions were prepared in PBS. Undiluted LAB was mixed with each of the C. albicans dilutions and undiluted C. albicans was mixed each of the LAB dilutions. Likewise, diluted C. albicans was mixed with diluted LAB. From both LAB and C. albicans dilutions, 200 $\mu$L were mixed in the wells of a 48 wells microtiter plate and incubated on a shaker at 200 rpm at room temperature. After 24 hours, it was observed if co-aggregation and auto-aggregation appeared in the wells.
Formation of co-aggregation was scored visually from 1-5 using the following scale:

1: No aggregation
2: Visual initial aggregation
3: Formation of aggregates < 0.5 mm
4: Formation of aggregates > 0.5 mm and < 1 mm
5: Formation of aggregates > 1 mm

Example 4 Prevention of Candida biofilm

The effect of lactic acid bacteria on prevention of biofilm formation of Candida was determined as described in Gottschick et al. (2016) Screening of compounds against Candida biofilms. PLos One 11(4). doi.org/10.1371/journal.pone.0154086.

A pre-culture of Candida was prepared in YPD broth and incubated 24 hours at 37°. This pre-culture was diluted to OD600 = 0.05 in PBS for the final biofilm culture. The lactic acid bacteria were prepared in a similar manner, using MRS for the pre-culture, diluted to OD600 = 0.5 in PBS and mixed 1:1 before cultivation in Nunc™ Microwell™ 96-Well Microplates (Thermo Scientific). The mixture is removed from the well after 30 min, washed once with PBS to remove unattached cells and incubated in YPD for 20 hours at 37°C. Supernatant was removed, and biofilms were washed twice with sterile PBS buffer. OD600 measured as compared to control with no biofilm and control biofilm of each microorganism grown as a single biofilm culture.

Inhibition of biofilm formation by lactic acid bacteria (LAB) was determined as:

((OD600[biofilm of Candida] - OD600[mixed biofilm culture of LAB test strain and Candida])/OD600 [biofilm of Candida]) x 100%

The selected strains were compared to commercially available consumer products.

Product A: GynoLact available in Denmark by Vitabalans comprising 3 Lactobacillus strains (L. acidophilis, L. casei, L. rhamnosus). Pre-culture for test was prepared by breaking one tablet into MRS medium and incubate anaerobically overnight at 37°C.

Product B: Vivag Capsule available in Denmark by Orkla Care A/S comprising 2 Lactobacillus strains (L. gasseri EB01TM and L. rhamnosus PB01TM). Pre-culture for test was prepared by breaking one tablet into MRS medium and incubate anaerobically overnight at 37°C.

Each product was prepared for the experiment similar to Candida and LAB strains.

The selected LAB strains have a significant better ability to prevent biofilm formation by Candida.

Example 5 Well diffusion assay

Overnight cultures of LAB and C. albicans were prepared. LAB was grown in MRS broth and Candida was grown in YPD broth at 37 °C. From each of the Candida culture with an OD600 of approximately 0.5, a 10^-1 dilution was prepared, and 1 mL was plated on top of Mueller-Hinton agar plates. Wells were made in the plates and 50 µL of the LAB cultures were transferred to the wells. After incubation overnight at 37 °C, inhibition zones around the wells were examined.

Example 6 hydrogen peroxide formation

To investigate whether the LAB strains produced hydrogen peroxide ($H_2O_2$), a hydrogen peroxide assay was performed according to methods described by Pendharkar et al. (2013). MRS agar plates containing 0.25 mg/mL 3,3', 5,5'-tetramethylbenzidine (TMB) and 0.01 mg/mL horseradish peroxidase were made. Both the TMB and the peroxidase were added to the agar after autoclavation. A magnet stirrer was used to stir the bottle with the TMB and the peroxidase before the agar was poured into plates. From overnight LAB cultures, 20 µL were spotted on top of the agar. The plates were incubated anaerobically at 37 °C for three days and were then exposed to air for 30 minutes. $H_2O_2$ production was observed as blue coloured colonies. The production of $H_2O_2$ was given scores between 0 and 2.

Example 7 Challenge assay with LAB Cell Free Supernatants

C. albicans strain and LAB were incubated overnight at 37 °C. C. albicans was incubated in YPD broth and the LAB strains were incubated in MRS broth. Cell free supernatants (CFS) were made from the LAB cultures by the use of a syringe and a 0.2 µm filter. The C. albicans culture with an OD600 of approximately 0.5 was diluted 10^-2. From each of the C. albicans dilutions, 100 µL was challenged with 100 µL CFS dilutions of 100, 75, 50, 25, 10 and 0 % CFS in a 96 wells microtiter plate. The CFS dilutions were made using MRS broth. The plate was incubated in an oCelloscope at 37 °C using contrast phase microscopy and image analysis as growth measurements (oCelluScope, BioSense Solution, Denmark). Growth kinetics measurements were performed every 20th minute for 8 hours.

Example 8 Co-culture assay

LAB and C. albicans were grown overnight at 37 °C. LAB was grown in MRS broth and C. albicans was grown in YPD broth. All cultures were adjusted to an OD600 of approximately 0.4. From 4 mL of each culture, bacteria were harvested by centrifugation at 4500 rpm for 10 minutes. The supernatant was discharged, and the pellet was resuspended in 2 mL PBS and then re-centrifugated at 4500 rpm for 10 minutes. After discharging the supernatant, the pellet was re-suspended in 4 mL of PBS (4.5 mL for C. albicans). From these solutions, 0.5 mL was transferred to 25 mL of BHI broth. C. albicans was mixed with each of the LAB strains and monocultures of LAB and C. albicans were made as well. After 0, 6, and 24 hours, serial dilutions were made and plated on BHI plates for enumeration of CFU. The mixed cultures and the C. albicans monocultures were plated on BHI agar plates supplemented with 32 µg/mL chloramphenicol to allow for the growth of C. albicans, but not LAB.

Results

All LAB isolated as described in example 1 were screened using the methods as described in examples 2-8, and two strains were identified as being antimicrobial against C. albicans in all the used methods.
The two identified strains were deposited on December 14th 2020 by Lactobio ApS, Copenhagen, Denmark with the German Collection of Microorganisms and Cell Cultures GmbH(DSMZ). Strains were deposited as followed:

Pediococcus pentosaceus LB606R deposited as DSM 33730
Lactiplantibacillus plantarum LB679R deposited as DSM 33731

Lactiplantibacillus plantarum was named according to taxonomy change as released on15th April 2020 by International Journal of Systematic and Evolutionary Microbiology. Former known as Lactobacillus plantarum.

Table 1: Spot on lawn and diffusion assay, inhibition zones are measured in mm.

|  | Spot on lawn (Diameter in mm) | | Well diffusion (Radius mm) | |
|---|---|---|---|---|
|  | LB606R | LB679R | LB606R | LB679R |
| C. albicans, strain P57055 | 11 +/- 2 | 14 +/- 3 | 4 +/- 1 | 5 +/- 1 |
| C. albicans, strain L26 | 13 +/- 3 | 16 +/- 2 | 2 +/- 1 | 3 +/- 1 |
| C. albicans, strain 19F | 8 +/- 2 | 13 +/- 2 | 3 +/- 1 | 3 +/- 1 |

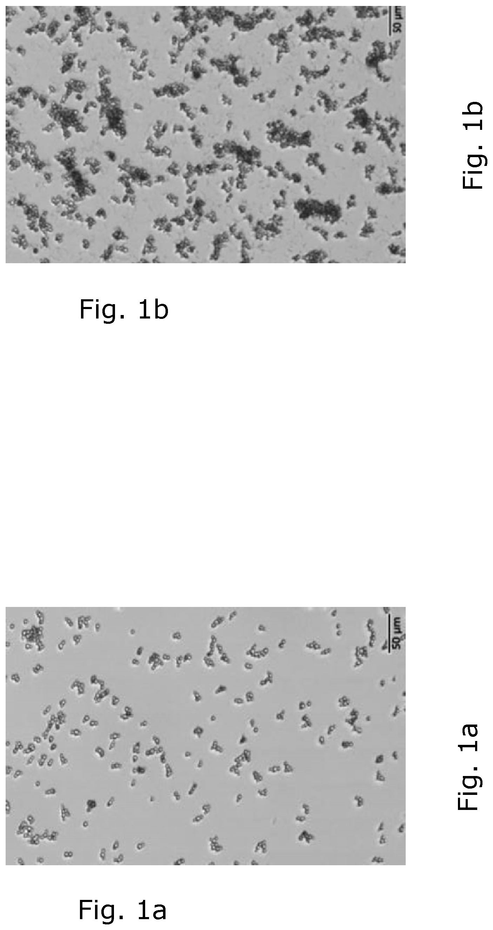

Co-aggregation was for both strain LB606R and LB679R determined to be 3-4. Example shown in Figure 1 for co-aggregation between C. albicans strain L26 and LB606R.

Table 2: Reduction of C. albicans biofilm (strain L26 and 19F).

|  | 16S RNA identification (Sanger) | Deposit number | Reduction of biofilm (%) | |
|---|---|---|---|---|
| strain |  |  | L26 | 19F |
| LB606R | Pediococcus pentosaceus | DSM33730 | 81 +/-8 | 75 +/-12 |
| LB679R | Lactiplantibacillus plantarum | DSM33731 | 87 +/-6 | 89 +/- 9 |
| Product A | mix | Commercial product | 6 +/-6 | 10 +/- 6 |
| Product B | mix | Commercial product | 13 +/-20 | 17 +/- 8 |

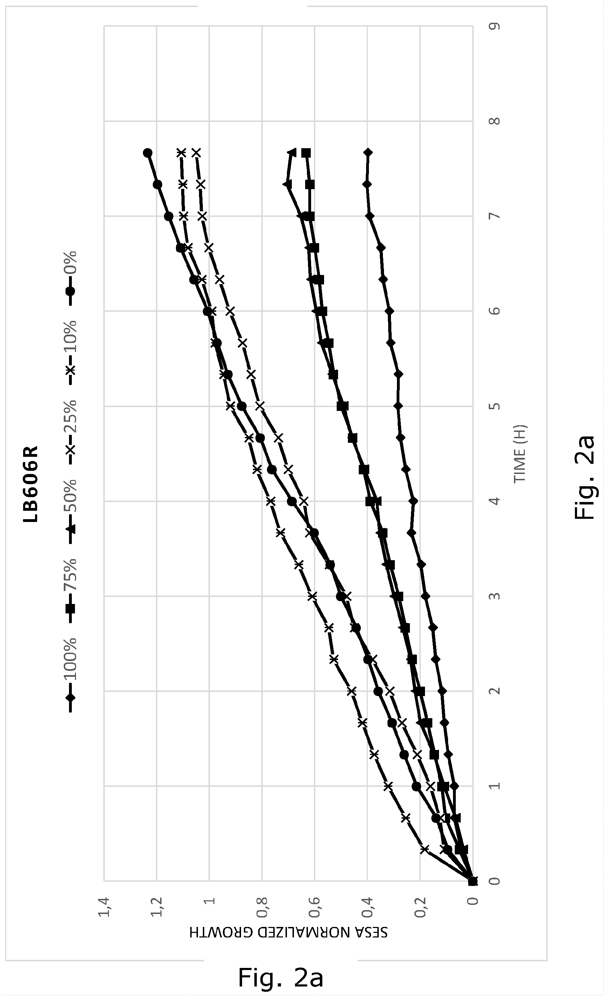

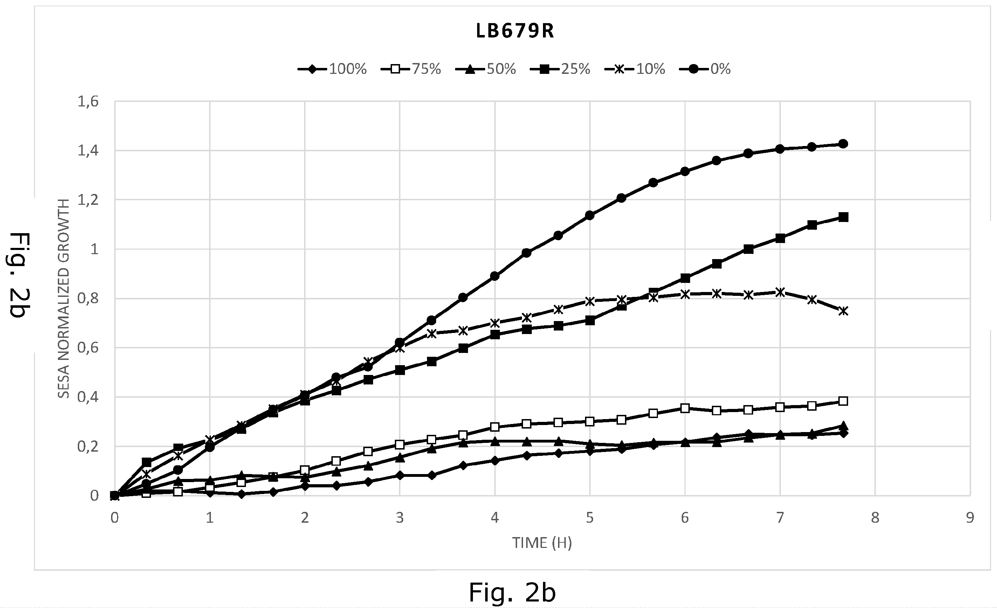

Cell free supernatant of both LB606R and LB679R were able to significantly inhibit growth of C. albicans diluted to a concentration of 25%. Data shown for C. albicans strain L26 in figure 2a and 2b. In co-culture assay both strains were able to out-compete all 3 test strains of Candida albicans.
Figure 1: Co-aggregation of C. albicans strain L26 and LB606R. The control of C. albicans L26 Fig 1a shown with the combination and co-aggregation with LB606R (Fig. 1b).
Figure 2: Growth inhibition by CFS of LB606R (Fig. 2a) and LB679R (Fig 2b) against growth of C. albicans strain L26.

**References**

Silva, S., M. Negri, M. Henriques,R. Oliveira, DW. Williams and J. Azeredo (2012) Candida glabrata, Candida parapsilosis and Candida tropicalis: biology, epidemiology, pathogenicity and antifungal resistance. FEMS Microbiology Reviews, Volume 36 (2): 288-305, https://doi.org/10.1111/j.1574-6976.2011.00278.x

Pfaller, MA, JD Diekema (2007) Epidemiology of invasive candidiasis: a persistent public health problem. Clin Microbiol Rev 20: 133-163.

Luo, G., Samaranayake, LP, and JY. Yau (2001) Candida species exhibit differential in vitro hemolytic activities. J. Clin. Microbiol. 39:2971-2974.

Negri M et al 2010. Mycophatologia 169: 175-182. Doi:10.1007/s11046-009-9246-0

Dowarah, R., et al. (2018) Selection and characterization of probiotic lactic acid bacteria and its impact on growth, nutrient digestibility, health and antioxidant status in weaned piglets. PLoS ONE, 13(3)

Kwak, Y-K. Et al. (2016) Persistence of Lactobacilli in postmenopausal women - a double-blind, randomized, pilot study. Gynecol. Obstet Invest 82: 144-150. Doi: 10.1159/000446946

Gottschick et al. (2016) Screening of compounds against Gardnerella vaginalis biofilms. PLos One 11(4). doi.org/10.1371/journal.pone.0154086

Khare, A., & Tavazoie, S. (2015). Multifactorial Competition and Resistance in a Two-Species Bacterial System. PLoS Genetics, 11(12), 1-21.

Marshall, V.M. (1979) J. Appl. Bacteriol. 47 pp 327-328. doi.org/10.1111/j.1365-2672.1979.tb01762.x

**Claims**

1. A composition comprising *Lactiplantibacillus plantarum* LB 679R, deposited under the deposit assession number DSM 33731.

2. The composition according to claim 1, wherein the composition inhibits biofilm formation.

3. The composition according to anyone of the preceding claims, wherein the composition is formulated for oral use, topical use, anal use or genital use.

4. The composition according to anyone of the preceding claims, wherein the composition further comprises one or more prebiotic.

5. The composition according to anyone of the preceding claims, wherein the *Lactiplantibacillus plantarum* LB 679R, deposited under the deposit assession number DSM 33731 is provided as viable cells, as non-viable cells, as a lysate, or as a supernatant.

6. The composition according to anyone of the preceding claims, wherein the *Lactiplantibacillus plantarum* LB 679R, deposited under the deposit assession number DSM 33731is stabilized in freeze dried, lyophilized or microencapsulated forms.

7. The composition according to claim 5, wherein the non-viable cells are provided as a lysate, as a fraction, as a ferment, as metabolites, or as an extract of one of the *Lactiplantibacillus* strains.

8. The composition according to any one of claims 5 or 7, wherein the lysate includes the cell wall or cell wall constituents including binding receptors.

9. The composition according to anyone of the preceding claims, wherein the *Lactiplantibacillus plantarum* LB 679R, deposited under the deposit assession number DSM 33731 is an isolated *Lactiplantibacillus plantarum* LB 679R, deposited under the deposit assession number DSM 33731.

10. An isolated bacterial strain selected from *Lactiplantibacillus plantarum* LB 679R, deposited under the deposit assession number DSM 33731.

11. A composition comprising one or more *Lactiplantibacillus plantarum* strain, wherein the composition is a topical composition to be applied on the skin.

12. A composition comprising one or more bacterial strains selected from one or more *Lactiplantibacillus plantarum* strain for use in the prevention and/or treatment of *Candida albicans* candidiasis in a human or in an animal, wherein the composition is for topical use to be applied on the skin.

13. A composition comprising one or more bacterial strains selected from one or more *Lactiplantibacillus plantarum* strain for the therapeutic use in the prevention, inhibition, or treatment of biofilm formation, wherein the composition is a topical composition to be applied on the skin, wherein the biofilm formation is a *Candida albicans* biofilm formation.

14. The composition according to anyone of claims 11-13, wherein the composition is formulated into a suspension, gel, cream, lotion, powder, capsule, ointment, oil, microencapsulated product.

15. A method for preventing biofilm formation in an environment, wherein the biofilm comprises Candida albicans comprising the steps of: administering to the environment an effective amount of a lactic acid bacteria selected from one or more *Lactiplantibacillus plantarum* strain with antimicrobial activity, wherein the environment is a home, workplace, laboratory, industrial environment, aquatic environment, medical device, or dental device.

Fig. 1b

Fig. 1a

Fig. 2a

Fig. 2b

EP 4 768 035 A2

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description

- EP 2823822 A

- FI 92498

### Non-patent literature cited in the description

- **DOWARAH, R. et al.** Selection and characterization of probiotic lactic acid bacteria and its impact on growth, nutrient digestibility, health and antioxidant status in weaned piglets. *PLoS ONE*, 2018, vol. 13 (3)
- **KHARE, A.** ; **TAVAZOIE, S.** Multifactorial Competition and Resistance in a Two-Species Bacterial System. *PLoS Genetics*, 2015, vol. 11 (12), 1-21
- **CISAR, J. O. et al.** Specificity of Coaggregation Reactions between Human Oral Streptococci and Strains of Actinomyces Viscosus or Actinomyces Naeslundii.. *Infection and Immunity*, 1979, vol. 24 (3), 742-52
- **GOTTSCHICK et al.** Screening of compounds against Candida biofilms. *PLos One*, 2016, vol. 11 (4)
- *International Journal of Systematic and Evolutionary Microbiology*, 15 April 2020

- **SILVA, S.** ; **M. NEGRI** ; **M. HENRIQUES** ; **R. OLIVEIRA** ; **DW. WILLIAMS** ; **J. AZEREDO**. Candida glabrata, Candida parapsilosis and Candida tropicalis: biology, epidemiology, pathogenicity and antifungal resistance. *FEMS Microbiology Reviews*, 2012, vol. 36 (2), 288-305, https://doi.org/10.1111/j.1574-6976.2011.00278.x
- **PFALLER, MA** ; **JD DIEKEMA**. Epidemiology of invasive candidiasis: a persistent public health problem. *Clin Microbiol Rev*, 2007, vol. 20, 133-163
- **LUO, G.** ; **SAMARANAYAKE, LP** ; **JY. YAU**. Candida species exhibit differential in vitro hemolytic activities. *J. Clin. Microbiol.*, 2001, vol. 39, 2971-2974
- **NEGRI M et al.** *Mycophatologia*, 2010, vol. 169, 175-182
- **KWAK, Y-K. et al.** Persistence of Lactobacilli in postmenopausal women - a double-blind, randomized, pilot study. *Gynecol. Obstet Invest*, 2016, vol. 82, 144-150
- **GOTTSCHICK et al.** Screening of compounds against Gardnerella vaginalis biofilms. *PLos One*, 2016, vol. 11 (4)
- **MARSHALL, V.M.** *J. Appl. Bacteriol.*, 1979, vol. 47, 327-328